# EUROPÄISCHE PATENTSCHRIFT

(11) **EP 0 966 273 B1**
(45) Veröffentlichungstag und Bekanntmachung des Hinweises auf die Patenterteilung: **19.05.2004**
(21) Anmeldenummer: 98906935.6
(22) Anmeldetag: 09.02.1998
(51) Int. Cl.: A61K 9/70

(54) **TRANSDERMALES ODER TOPISCHES PFLASTERSYSTEM MIT POLYACRYLATMATRIX MIT VERBESSERTEN PHYSIKALISCHEN EIGENSCHAFTEN**
TRANSDERMAL OR TOPICAL PLASTER SYSTEM WITH A POLYACRYLATE MATRIX WITH IMPROVED PHYSICAL PROPERTIES
SYSTEME A EMPLATRE TRANSDERMIQUE OU TOPIQUE AVEC MATRICE AU POLYACRYLATE A PROPRIETES PHYSIQUES AMELIOREES

(30) Priorität: 21.02.1997 DE 19706824
(43) Veröffentlichungstag der Anmeldung: 29.12.1999
(73) Patentinhaber: LTS Lohmann Therapie-Systeme AG, 56626 Andernach (DE)
(72) Erfinder: MÜLLER, Walter, D-56564 Neuwied (DE)
(74) Vertreter: Flaccus, Rolf-Dieter, Dr.
(86) Internationale Anmeldenummer: PCT/EP1998/000685
(87) Internationale Veröffentlichungsnummer: WO 1998/036740

(56) Entgegenhaltungen:
- EP-A- 0 464 573
- WO-A-86/00814
- WO-A-98/25591
- CHEMICAL ABSTRACTS, vol. 114, no. 20, 20. Mai 1991 Columbus, Ohio, US; abstract no. 192597, KISHI TAKASHI: "Therapeutic tapes containing multiple layers of adhesives" XP002078921 & DATABASE WPI Section Ch, Week 9036 Derwent Publications Ltd., London, GB; Class A96, AN 90-272632 & JP 02 193915 A (SEKISUI CHEM CO LTD) , 31. Juli 1990 & PATENT ABSTRACTS OF JAPAN vol. 14, no. 470, 15. Oktober 1990 & JP 02 193915 A (SEKISUI CHEM CO LTD), 31. Juli 1990

## Beschreibung

Die Erfindung bezieht sich auf ein transdermales oder topisches Pflästersystem mit einer Rückschicht, einer wirkstoffhaltigen und selbstklebenden Matrix auf der Basis von vernetzbarem Polyacrylat als Grundpolymer und einer wiederentfernbaren Schutzfolie.

Transdermale therapeutische Systeme (TTS) haben sich mittlerweile aufgrund der besonderen Vorteile dieser Darreichungsform für bestimmte Wirkstoffe ihren festen Platz in der Therapie einiger Krankheiten erobert. Die auf dem Markt befindlichen Pflaster unterscheiden sich voneinander bezüglich ihres technischen Aufbaus, der eingesetzten Wirkstoffe, Hilfsstoffe und Polymere.

Bezüglich des technischen Aufbaus sind es im wesentlichen zwei Pflastersysteme, die den Markt heute dominieren. Es sind dies die sogenannten Reservoirsysteme und die sogenannten Matrixsysteme.
Die Reservoirsysteme bestehen typischerweise aus einem Beutel, der mit einer flüssigen Zubereitung des Wirkstoffs befüllt ist. Die eine Seite des Beutels besteht dabei aus einer zumindest für den Wirkstoff durchlässigen Membran und ist üblicherweise noch mit einem geeigneten Kleber versehen.
Bei den Matrixsystemen ist der Wirkstoff in einer Polymerformulierung (Matrix) eingearbeitet, die bevorzugt auch selbstklebend ist. Deshalb bestehen Matrixsysteme im einfachsten Fall aus einer flexiblen Folie oder textilem Gewebe, die die Rückschicht des Pflasters bilden, einer oder mehreren wirkstoffhaltigen, bevorzugt selbstklebenden Matrixschichten und einer vor Gebrauch zu entfernenden Schutzschicht.

Matrixsysteme stellen gewissermaßen die zweite Generation der transdermalen Systeme dar und werden bei Neuentwicklungen bevorzugt angestrebt. Sie bieten gegenüber den Reservoirsystemen den Vorteil eines höheren Tragekomforts und einer höheren inhärenten Sicherheit, da ein Dosedumping durch ein Auslaufen des Reservoirs nicht befürchtet werden muß. Solche Matrixsysteme werden auch als sogenannte topische Systeme verwendet, bei denen der Wirkstoff seine Wirkung bevorzugt an der Applikationsstelle entfaltet und die systemische Verfügbarkeit nicht angestrebt wird.

Die bevorzugte Polymerklasse für die Formulierung der Matrix sind dabei Polyacrylatkleber, da sie über ein gutes Klebevermögen auf der Haut verfügen, hypoallergen sind und in vielfältiger Ausführung mit unterschiedlichen Eigenschaften bezüglich ihrer physikalischen und chemischen Eigenschaften verfügbar sind.

Die Entwicklung von selbstklebenden Matrixsystemen wird dadurch erschwert, daß in dem Kleber sowohl der Wirkstoff als auch alle sonstigen Hilfsstoffe wie z.B. Permeationsverbesserer eingearbeitet sein müssen und dadurch die Eigenschaften des Klebers oftmals verschlechtert werden. Insbesondere die Kohäsion des Klebers ist davon betroffen, was sich dann während ihrer Lagerung in dem sogenannten kalten Fluß bzw. darin äußert, daß beim Entfernen des Pflasters von der Haut Kleberreste an der Applikationsstelle verbleiben.

Unter kaltem Fluß versteht man dabei, daß die Klebermatrix zwischen Rückschicht und Schutzschicht bei Lagerung wie eine sehr viskose Flüssigkeit zu fließen beginnt und letztendlich der Kleber nicht mehr vollständig von der Rückschicht abgedeckt ist. Dadurch kann z.B. das Pflaster mit dem Primärpackbehältnis, das gewöhnlich aus einem Beutel aus heißgesiegeltem Packstofflaminat besteht, verkleben und dadurch unbrauchbar werden.
Besonders evident werden diese Probleme, wenn die Konzentration an im Kleber gelösten weichmachenden Wirk- und/oder Hilfsstoffen mehr als 10 Gewichtsprozent beträgt und das Gesamtbeschichtungsgewicht der Matrix 50g/m² übersteigt.

Als Mittel, diesen kalten Fluß bei Polyacrylatklebern zu unterdrücken, wird in der Patentanmeldung WO 86/00814 eine Vernetzung des Polymeren in der Weise vorgesehen, daß die weichmachende Wirkung der Inhaltsstoffe durch Vernetzung kompensiert wird. Dabei ist allerdings zu beachten, daß die Klebrigkeit des Pflasters bei zu starker Vernetzung so stark abnimmt, daß die Haftung auf der Haut ungenügend wird.
Die Herstellung eines gut klebenden Pflasters mit einem akzeptablen kalten Fluß bleibt mithin problematisch.

Das gleiche gilt für die in dem Patent DE 40 20 144 vorgeschlagene Lösung, der Matrix ein filmbildendes nichtklebendes Polymer zuzusetzen.

Das Dokument WO-A-98 25591 beschreibt ein Pflastersystem mit einer Rückschicht, einer selbstklebenden Matrix mit zwei Schichten und einer Schutzfolie. Die Konzentration des Vernetzers in der der Haut zugewandten Matrixschicht ist in WO-A-98 25591 geringer als die Konzentration des Vernetzers in der darüber liegenden Matrixschicht.

Die Lösung des Problems, die Kohäsion der Matrix zu verbessern, ohne die Klebkraft auf der Haut zu reduzieren, wurde nun überraschenderweise darin gefunden, die Matrix aus mindestens zwei Schichten aufzubauen, die insbesondere die gleiche Polymerzusammensetzung und die gleiche Konzentration an gelösten Inhaltsstoffen aufweisen, wobei die der Haut abgewandte(n) Schicht(en) einen zur Vermeidung von kaltem Fluß genügenden Grad an Vernetzung besitzen und die der Haut zugewandte Schicht einen Vernetzungsgrad aufweist, der eine genügende Hauthaftung garantiert.

Demgemäß ist das erfindungsgemäße Pfastersystem der eingangs genannten Art im wesentlichen gekennzeichnet durch eine mindestens zweischichtige Matrix mit einem Vernetzungsgrad der der Haut zugewandten Schicht, der einer genügenden Hauthaftung derselben angepaßt und geringer ist als der Vernetzungsgrad der darüberliegenden Schicht bzw. Schichten.

Das Beschichtungsgewicht der Hautkontaktschicht liegt dabei bevorzugt bei 10-30 g/m². Bei einer solchen Dicke ist der kalte Fluß der Schicht vernachlässigbar gering, während der Vernetzungsgrad eine exzellente Hauthaftung ergibt.

Weitere Besonderheiten der Erfindung ergeben sich aus den Patentansprüchen und der nachfolgenden Beschreibung.

Die Vernetzung der Matrixschichten in unterschielichem Ausmaß kann in an sich bekannter Weise erfolgen, wie z.B. durch
Zusatz von Metallchelaten wie z.B. Aluminiumacetylacetonat oder Titanacetylacetonat,
chemische Vernetzung mit reaktiven Reagenzien wie z.B. Melamin,
Vernetzung durch Elektronenbestrahlung,
Bestrahlung mit UV-Licht bei Klebern mit entsprechenden dafür geeigneten funktionellen Gruppen.

Besonderer Nutzen ergibt sich erfindungsgemäß aus der Erkenntnis, daß zwei wichtige und für die Leistung des TTS entscheidende Parameter praktisch nicht durch den Grad der Vernetzung beeinfluß werden. Es sind dies
a) die Sättigungslöslichkeit von allen niedermolekularen Substanzen und damit auch des Wirkstoffes und
b) der Diffusionskoeffizient von niedermolekularen Substanzen,
wodurch die konkrete Erarbeitung eines speziellen Pflastersystems eine erhebliche Verbesserung erfährt.

Die Sättigungslöslichkeit und damit die thermodynamische Aktivität des Wirkstoffs bei gegebener Konzentration wird deshalb nicht beeinflußt, weil im allgemeinen nur ein sehr kleiner Teil der funktionellen und für die Vernetzung notwendigen Gruppen des Polymeren an der Vernetzüngsreaktion teilnimmt. Der Beitrag der funktionellen Gruppen zur Polarität des Polymeren und damit auch zu dessen Lösevermögen bleibt also weitgehend erhalten.
Der Diffusionskoeffizient von niedermolekularen Verbindungen bleibt von dem Grad der Vernetzung weitgehend unbeeinflußt, weil für die Diffusion nur die Mikroviskosität des Polymeren in der unmittelbaren Umgebung der diffundierenden Verbindung von Bedeutung ist. Diese Mikroviskosität ist jedoch, bedingt durch die große Länge der Polymermoleküle, bei allen praktisch notwendigen Vernetzungsgraden nahezu unbeeinflußt.

Ist also eine auf einem zur Vernetzung befähigten selbstklebenden Polyacrylat beruhende Matrixformulierung gefunden, die es erlaubt, eine für die praktische Anwendung ausreichende Menge an Wirkstoff durch die Haut abzugeben, dann kann ohne weiteres ein erfindungsgemäßer Aufbau dieser Matrix aus mehreren Schichten mit unterschiedlichen Vernetzungsgraden unter Optimierung von Dicke, Kohäsion und Klebkraft vorgesehen werden, ohne daß die Permeationsleistung des Systems dadurch beeinflußt wird.

Dies bedeutet eine signifikante Vereinfachung für die Entwicklung von Matrixsystemen. Eine Vereinfachung deshalb, weil Probleme mit der Klebkraft und der Kohäsion oft erst dann evident werden, wenn schon erste Klinikversuche und Stabilitätsstudien durchgeführt wurden. Eine in diesem Stadium notwendige Umformulierung der Matrix bezüglich ihrer Zusammensetzung bedeutete bisher, daß die Ergebnisse dieser Klinikversuche und der Stabilitätsstudien in Frage gestellt wurden. Eine Optimierung der Matrix im Sinne dieser Erfindung bedeutet, daß die Ergebnisse der Klinikversuche voll übernommen werden können und keine zusätzlichen Stabilitätsrisiken erwartet werden müssen. Dies wird anhand der weiter unten angegebenen tabellarischen Gegenüberstellung von Permeationsdaten eines erfindungsgemäßen Pflasters mit zweischichtiger Matrix und einem herkömmlichen Pflaster deutlich.

Es folgen Beispiele zur Erläuterung der Erfindung.

### Beispiel 1: Pflaster mit Scopolamin als Wirkstoff

Zu 100 g einer Polyacrylatkleberlösung (Durotak 326-1753) mit einem Feststoffgehalt von 50 % werden 12,5 g Oleylalkohol und 12,5 g Scopolamin gegeben und die Lösung durch Rühren homogenisiert.

Zu 80 g dieser Lösung werden 13,6 g einer 4-prozentigen Lösung von Aluminiumacetylacetonat in Ethylacetat gegeben und die Lösung durch Rühren homogenisiert.
Die resultierende Lösung ist die Klebermasse A.

Zu 20 g der wirkstoffhaltigen Kleberlösung werden 0,85 g der 4-prozentigen Lösung von Aluminiumacetylacetonat in Ethylacetat gegeben und auch die Lösungen durch Rühren homogenisiert.
Die resultierende Lösung ist die Klebermasse B mit geringerer Metallchelatkonzentration.

Die Klebermasse A wird mittels einer Rakel in einer Dicke auf eine silikonisierte Polyesterfolie gestrichen, daß nach dem Entfernen der Lösemittel ein Beschichtungsgewicht von 80 g/m² resultiert. Die getrocknete Masse wird mit einer 25 µm dicken Polyesterfolie kaschiert.

Die Klebermasse B wird mittels einer Rakel in einer Dicke auf eine silikonisierte Polyesterfolie gestrichen, daß nach dem Entfernen der Lösemittel ein Beschichtungsgewicht von 20 g/m² resultiert.

Die getrocknete Masse wird mit dem oben beschriebenen getrockneten Film kaschiert, nachdem die silikonisierte Folie entfernt wurde.
Aus dem so hergestellten aus der 25 µm dicken Polyesterfolie, zwei unterschiedlich vernetzten Matrixschichten und einer silikonisierten Polyesterfolie bestehenden Vierschichtlaminat können nun die fertigen Pflaster gestanzt werden.

Das so hergestellte Pflaster wurde einer Permeationsstudie unter Verwendung einer 0,127 mm dicken Silikonmembran als Modellmembran für menschliche Haut unterworfen, um zu zeigen, daß sich ein zweischichtiges Matrixsystem mit unterschiedlichem Vernetzungsgrad im Sinne dieser Erfindung praktisch nicht von einem herkömmlichen einschichtigen Matrixsystem (nur aus Klebermasse A in 100 g/m² Dicke) bezüglich der Permeationsrate des eingearbeiteten Wirkstoffs unterscheidet.
Die künstliche Membran anstelle von Haut wurde deshalb gewählt, weil es die variabilität der natürlichen Hautpräparate schwer macht, kleine Unterschiede zwischen verschiedenen Pflasterformulierungen zu zeigen. Ansonsten wurde der Versuch mit den in der Literatur beschriebenen und dem Fachmann bekannten Franz-Diffusionszellen durchgeführt. Die Ergebnisse dieses Versuchs sind in der nachfolgenden Tabelle wiedergegeben.
Bei beiden Systemen war die Menge des eingearbeiteten Wirkstoffs und die Wirkstoffkonzentration gleich. Bei dem zweischichtigen System gemäß der Erfindung ist jedoch die Klebkraft und damit auch die Klebkraft auf der Haut signifikant verbessert.

| | Permeierte Menge an Scopolamin Base [µg/cm²] nach | | | | | | | | |
|---|---|---|---|---|---|---|---|---|---|
| | 0 h | 2 h | 4 h | 6 h | 8 h | 24 h | 30 h | 48 h | 72 h |
| Einschicht Pflaster | 0 | 14,1 | 30,4 | 44,8 | 59,3 | 152,9 | 185,4 | 263,2 | 355,8 |
| Zweischicht Pflaster | 0 | 17,5 | 33,8 | 46,0 | 61,0 | 150,0 | 181,7 | 258,7 | 356,8 |

### Beispiel 2 : Pflaster mit Ketoprofen als Wirkstoff

Zu 100 g einer Polyacrylatkleberlösung (Durotak 326-1051) mit einem Feststoffgehalt von 50 % werden 6 g Ölsäure und 15 g Tiaprofensäure gegeben und die Lösung solange mechanisch gerührt bis alle Tiaprofensäure gelöst ist.

Zu 80 g dieser Lösung werden 15 g einer 4-prozentigen Lösung von Aluminiumacetylacetonat in Ethylacetat gegeben und die Lösung durch Rühren homogenisiert.
Die resultierende Lösung ist die Klebermasse A.

Zu 20 g der wirkstoffhaltigen Kleberlösung werden 2 g der 4-prozentigen Lösung von Aluminiumacetylacetonat in Ethylacetat gegeben und auch die Lösung durch Rühren homogenisiert.
Die resultierende Lösung ist die Klebermasse B.

Die Klebermasse A wird mittels einer Rakel in einer Dicke auf eine silikonisierte Polyesterfolie gestrichen, daß nach dem Entfernen der Lösemittel ein Beschichtungsgewicht von 100 g/m² resultiert. Die getrocknete Masse wird mit einem querelastischen Gewebe aus Viskose kaschiert. Die Klebermasse B wird mittels einer Rakel in einer Dicke auf eine silikonisierte Polyesterfolie gestrichen, daß nach dem Entfernen der Lösemittel ein Beschichtungsgewicht von 25 g/m² resultiert.
Die getrocknete Masse wird mit dem oben beschriebenen getrockneten Film kaschiert, nachdem die silikonisierte Folie entfernt wurde.
Aus dem so hergestellten aus dem Viskosegewebe, zwei unterschiedlich vernetzten Matrixschichten und einer silikonisierten Polyesterfolie bestehenden Vierschichtlaminat können nun die fertigen Pflaster gestanzt werden.

## Patentansprüche

1. Transdermales oder topisches Pflastersystem mit einer Rückschicht, einer wirkstoffhaltigen und selbstklebenden Matrix auf der Basis von vernetzbarem Polyacrylat als Grundpolymer und einer wiederentfernbaren Schutzfolie, **gekennzeichnet durch** eine mindestens zweischichtige Matrix, wobei die der Haut zugewandte Matrixschicht
a) einen Vernetzungsgrad aufweist, der geringer ist als der Vernetzungsgrad der darüber liegenden Matrixschicht und
b) eine Hauthaftung gewährleistet.

2. Pflastersystem nach Anspruch 1, **gekennzeichnet durch** eine Schichtdicke der Hautkontaktschicht entsprechend 10-30 g/m².

3. Pflastersystem nach Anspruch 1 oder 2, **gekennzeichnet durch** eine Vernetzung **durch** entsprechende Metallchelatmengen in Hydroxyl- und/oder Carboxylgruppen enthaltenden Acrylatpolymeren.

4. Pflastersystem nach Anspruch 1 oder 2, **gekennzeichnet durch** eine Vernetzung **durch** eine Titanacetylacetonatverbindung oder Aluminiumacetylacetonat.

5. Pflastersystem nach Anspruch 1 oder 2, **gekennzeichnet durch** eine Vernetzung **durch** Elektronenstrahleinwirkung oder UV-Bestrahlung.

6. Pflastersystem nach einem der vorangehenden Ansprüche, **dadurch gekennzeichnet, daß** die einzelnen Matrixschichten die gleiche Polymerzusammensetzung und die gleiche Konzentration an gelösten Inhaltsstoffen aufweisen.

7. Pflastersystem nach einem der vorangehenden Ansprüche, **gekennzeichnet durch** ein Gesamtbeschichtungsgewicht der Matrix von zumindest 50 g/m².

## Claims

1. Transdermal or topical plaster system with a backing layer, with an active ingredient-containing and self-adhesive matrix based on crosslinkable polyacrylate as base polymer and with a removable protecting layer, **characterized by** an at least two-layer matrix wherein the matrix layer facing the skin
a) has a degree of crosslinking that is lower than the degree of crosslinking of the matrix layer located above it, and
b) ensures adhesion to the skin.

2. Plaster system according to Claim 1, **characterized by** a layer thickness of the skin contact layer corresponding to 10-30 g/m².

3. Plaster system according to Claim 1 or 2, **characterized by** a crosslinking by appropriate amounts of metal chelate in hydroxyl and/or carboxyl group-containing acrylate polymers.

4. Plaster system according to Claim 1 or 2, **characterized by** a crosslinking by a titanium acetylacetonate compound or aluminium acetylacetonate.

5. Plaster system according to Claim 1 or 2, **characterized by** a crosslinking by the action of an electron beam or UV irradiation.

6. Plaster system according to any of the preceding claims, **characterized in that** the individual matrix layers have the same polymer composition and the same concentration of dissolved ingredients.

7. Plaster system according to any of the preceding claims, **characterized by** a total coating weight of the matrix of at least 50 g/m².

## Revendications

1. Système d'emplâtre transdermique ou topique comprenant une couche dorsale, une matrice autoadhésive et contenant une ou plusieurs substances actives, à base de polyacrylate réticulable à titre de polymère fondamental, et une couche de protection qui peut être retirée par la suite, **caractérisé par** une matrice contenant au moins deux couches, la couche matricielle tournée vers la peau
a) présentant un degré de réticulation qui est inférieur au degré de réticulation de la couche matricielle sus-jacente et
b) garantissant une adhérence cutanée.

2. Système d'emplâtre selon la revendication 1, **caractérisé par** une épaisseur de couche, en ce qui concerne la couche de mise en contact avec la peau, correspondant à 10-30 g/m².

3. Système d'emplâtre selon la revendication 1 ou 2, **caractérisé par** une réticulation mise en oeuvre par des quantités correspondantes de chélates métalliques dans des polymères d'acrylates contenant des groupes hydroxyle et/ou carboxyle.

4. Système d'emplâtre selon la revendication 1 ou 2, **caractérisé par** une réticulation mise en oeuvre via un composé d'acétylacétonate de titane ou via un acétylacétonate d'aluminium.

5. Système d'emplâtre selon la revendication 1 ou 2, **caractérisé par** une réticulation mise en oeuvre sous l'action de faisceaux électroniques ou par exposition à un rayonnement ultraviolet.

6. Système d'emplâtre selon l'une quelconque des revendications précédentes, **caractérisé en ce que** les couches matricielles individuelles présentent la même composition polymère et la même concentration de constituants dissous.

7. Système d'emplâtre selon l'une quelconque des revendications précédentes, **caractérisé par** un poids de la matrice, à titre de revêtement total, d'au moins 50 g/m².
